# FASCICULE DE BREVET EUROPEEN

(11) **EP 1 304 093 B1**
(45) Date de publication et mention de la délivrance du brevet: **12.10.2005**
(21) Numéro de dépôt: 02370043.8
(22) Date de dépôt: 21.10.2002
(51) Int. Cl.: A61F 2/46, A61B 5/103, G01L 1/00, A61B 19/00

(54) **Ancillaire pour la préparation de la mise en place d'une prothèse de genou**
Hilfsinstrument zur Vorbereitung des Einsetzens einer Knieprothese
Ancillary for preparing the implantation of a knee prosthesis

(30) Priorité: 22.10.2001 FR 0113631
(43) Date de publication de la demande: 23.04.2003
(73) Titulaire: Sofinordest, S.A.S., 67404 Illkirsch (FR); Gougeon, François, 59000 Lille (FR); Tillie, Bruno, 62000 Arras (FR)
(72) Inventeur: Gougeon, François, 59000 Lille (FR); Tillie, Bruno, 62000 Arras (FR)
(74) Mandataire: Hennion, Jean-Claude

(56) Documents cités:
- EP-A- 0 720 834
- EP-A- 0 979 636
- WO-A-92/17113
- WO-A-99/35972
- FR-A- 2 813 780
- GB-A- 2 306 653
- US-A- 4 779 349
- US-A- 5 213 112
- US-A- 5 470 354
- US-A- 5 656 785
- US-A- 5 997 545

## Description

L'invention se rapporte à un ancillaire pour un procédé de mesure de la balance ligamentaire et pour la préparation de la mise en place d'une prothèse de genou.

La pose d'une prothèse de genou présente plusieurs difficultés.

Le fémur et le tibia sont reliés par des ligaments interne et externe qu'il y a lieu de préserver lors de l'opération.

Ces ligaments exercent chacun une force de traction tendant à rapprocher l'extrémité inférieure du fémur et l'extrémité supérieure du tibia et également à maintenir le tibia et le fémur suivant une orientation préférentielle.

Les forces développées par ces ligaments dépendent également de la position angulaire du tibia par rapport au fémur à savoir si ce membre est en flexion ou en extension.

Selon chaque individu, il s'établit naturellement un équilibre des forces exercées par les ligaments, dit balance ligamentaire, qu'il est important de respecter.

Il faut donc pouvoir respecter à la fois la balance ligamentaire et éviter que la tension des ligaments après mise en place de la prothèse soit trop faible ou trop importante par rapport à la situation initiale.

A ce jour :
- lors de l'opération, le chirurgien met en tension les ligaments au moyen d'un tenseur ou de pinces, dites distracteurs, ceci afin d'écarter les deux parties de l'articulation pour ensuite procéder à la résection des parties fémorale et tibiale de l'os et
- c'est par la mesure de l'allongement des ligaments et avec son expérience que le chirurgien appréhende la balance ligamentaire.

Si l'opération de mise en place de cette prothèse ne respecte pas suffisamment cette balance ligamentaire, il se produit ensuite de nombreux désagréments pour le patient.

On connaît un dispositif de mesure d'une balance ligamentaire (US-5.470.354, WO-92/17113 et US-5.656.785) qui consiste, après pose des deux parties de la prothèse d'un genou, à insérer entre les deux parties de la prothèse un dispositif de mesure de la pression afin de déterminer cette balance ligamentaire et d'adapter la morphologie de la prothèse.

Cela nécessite de disposer de prothèses avec des moyens de réglage ce qui augmente le coût de ces prothèses.

Si on ne dispose pas de prothèses réglables, il faut soit recouper la partie tibiale, soit faire une rotation du plan d'appui du fémur.

Un autre problème réside dans le positionnement des coupes de résection.

En effet, la coupe tibiale doit être perpendiculaire à l'axe mécanique du tibia, axe généralement confondu avec l'axe médullaire.

Il en est de même avec le fémur mais cet axe mécanique est généralement incliné de quelques degrés de l'ordre de 3° à 11° par rapport à l'axe anatomique du fémur.

Le chirurgien utilise généralement des clichés de radiographies plans pour déterminer les coupes théoriques puis, au moyen de repères anatomiques, il positionne les gabarits de coupe.

Il commence par la coupe tibiale dont l'épaisseur ne doit pas être inférieure à une valeur prédéterminée, par exemple de huit millimètres.

Là encore, l'expérience du chirurgien est primordiale dans le choix des coupes.

En effet, le chirurgien doit retirer la partie de l'articulation détruite mais doit préserver le plus possible la partie saine.

On conçoit donc que le choix des coupes dépend pour beaucoup de l'expérience du chirurgien et non de paramètres physiques totalement maîtrisés et le choix de la coupe influence la balance ligamentaire.

L'invention a pour objet de remédier à ces inconvénients.

US-A-5,213,112 divulgue un ancillaire selon le préambule de la revendication 1.

Les inconvenients susmentionnés sont remédiés par un ancillaire comme défini par la revendication 1.

L'invention sera bien comprise à l'aide de la description ci-après faite à titre d'exemple non limitatif, en regard du dessin ci-annexé, qui représente schématiquement :

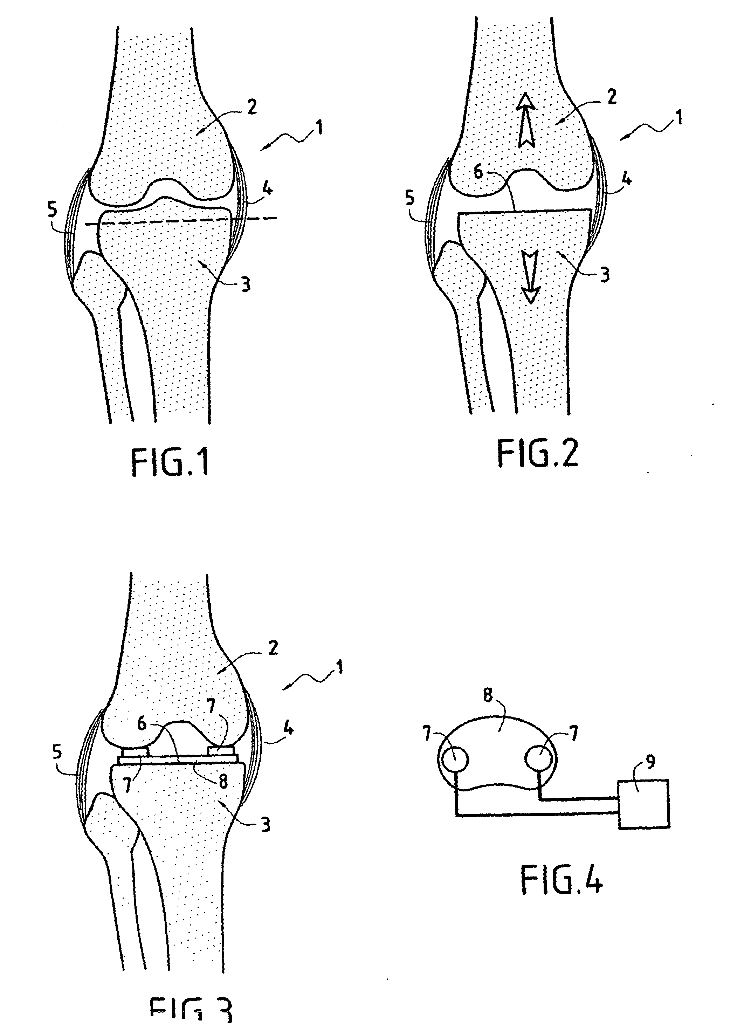

- figure 1 : vue de face d'une articulation avant résection,
- figure 2 : l'articulation après résection du plateau tibial,
- figure 3 : un ancillaire mis en place,
- figure 4 : l'ancillaire de la figure 3 en vue de dessus,
- figures 5 et 6 : différents ancillaires, objet de l'invention.

En se reportant au dessin, on voit une articulation 1 de genou.

Celle-ci se compose de deux parties à savoir une partie supérieure 2 qui est la base du fémur et une partie supérieure 3 qui est le haut du tibia.

Ces deux parties d'articulation sont reliées par le ligament 4 externe et le ligament 5 interne.

Ces deux ligaments exercent chacun une traction établissant un équilibre ligamentaire.

Pour la pose d'une prothèse de genou, on commence par définir l'emplacement de la coupe tibiale 6 et on utilise notamment un gabarit de coupe (non représenté) pour effectuer cette coupe.

Pour la définition de la coupe, après reconstitution en trois dimensions de la jambe du patient, un calculateur détermine la position de la coupe tibiale.

Après avoir procédé à la résection du plateau tibial, on insère le dispositif de mesure entre la coupe tibiale et on mesure la balance ligamentaire tant en position de flexion qu'en position jambe tendue.

Avantageusement, l'ancillaire pour la préparation de la mise en place d'une prothèse de genou comprend au moins un capteur 7 pour la mesure d'une grandeur physique proportionnelle à la force développée par les ligaments sur l'ancillaire disposé entre les deux parties de l'articulation.

Après ces mesures, les valeurs sont introduites à nouveau dans un calculateur qui va déterminer la position et l'orientation de la coupe fémorale en fonction de la morphologie de la prothèse et du patient.

Cela permet donc de garantir la préservation de la balance ligamentaire naturelle.

Dans une forme de réalisation, cet ancillaire comprend un support 8 pourvu de capteurs 7A de pression en vue d'être positionné entre la coupe tibiale et l'extrémité inférieure du fémur.

Le support se positionne sur la coupe tibiale et se présente sous la forme d'une plaque en appui sur la coupe tibiale et présentant sur sa face supérieure les capteurs 7A.

Par la mesure de la pression qui s'exerce tant en extension du membre qu'en flexion, on peut ainsi établir la balance ligamentaire.

Les capteurs de pression sont reliés à un dispositif 9 d'affichage de la mesure.

On peut utiliser des capteurs de pression de type mécanique ou de type piézorésistif.

Concernant les capteurs mécaniques, ce sont des capteurs de pression à manomètre.

Le principe de fonctionnement est le suivant : un tube métallique et recourbé communique avec l'enceinte dans laquelle se trouve le gaz dont on veut mesurer la pression.

Une des extrémités « l'entrée » est fixe ; la seconde est libre. Sous l'effet de la pression, le tube se déforme et l'extrémité libre du tube se déplace.

L'extrémité de ce tube en déformation étant relié (par l'intermédiaire d'un mécanisme à engrenage) à une aiguille, on peut alors lire la pression.

On peut également remplacer ce tube par une membrane qui se déplace en fonction de la pression. Un mécanisme relie alors une aiguille à cette membrane.

L'avantage d'utiliser un tel capteur est sa simplicité d'utilisation.

En effet, il ne nécessite aucun raccordement externe (à l'inverse d'une jauge d'extensométrie) puisqu'il fonctionne de manière autonome. De plus, la pression étant une fonction linéaire de la force appliquée sur le vérin, la conversion d'une pression en une force est facilitée.

Il suffit pour cela de graduer le cadran en Newtons en faisant la conversion. Et enfin, le système étant entièrement mécanique, il est possible de trouver un appareil résistant à des conditions sévères (135°C, 100% d'humidité).

Par contre, il augmente l'encombrement de l'extenseur.

Concernant les capteurs à membrane piézorésistive, avec ce type de capteur, on mesure la déformation d'une partie de l'enceinte renfermant le fluide dont on veut mesurer la pression.

On peut placer une jauge sur une membrane qui se déforme. La difficulté est de mesurer ces déformations sur une petite surface. Mais, il existe des constructeurs qui fabriquent des capteurs de pression miniature qui conviendraient au cadre de l'étude.

Il s'agit dans ce cas d'appareils munis de membrane piézorésistifs, dont la résistance varie en fonction de sa déformée. Il suffirait alors d'enfermer un capteur dans chacun des vérins pour mesurer la pression.

L'avantage de ce type de capteur par rapport au précédent, est qu'il est moins encombrant (dans le bloc opératoire au moins).

Par contre, il nécessite tout un appareillage à l'extérieur de l'enceinte (alimentation, acquisition des mesures). La lecture est plus facile (puisqu'il s'agit le plus souvent d'un affichage digital). Par contre, cet affichage ne pourra se faire qu'à l'extérieur de l'enceinte.

Dans une autre variante, on utilisera pour cette mesure le tenseur 10 utilisé pour écarter les deux parties de l'articulation.

Le tenseur comprend une potence 11 et un patin fémoral 12.

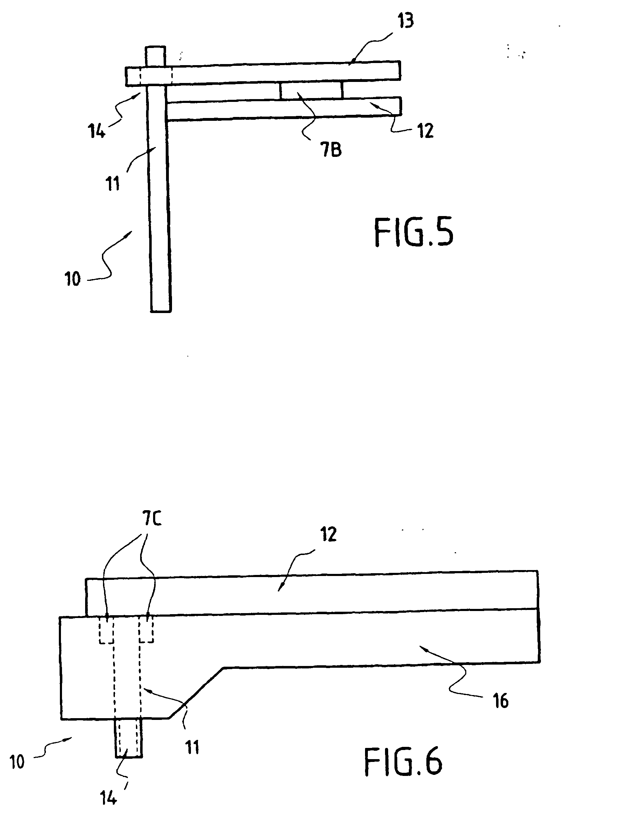

Dans une première variante de réalisation (figure 5), on ajoute au dessus du patin fémoral 12 existant un deuxième patin fémoral 13 libre en translation sur la potence et on positionne entre le patin fémoral existant et le second, un capteur 7B de force.

L'ancillaire comprend donc un capteur 7B de force disposé entre un patin fémoral 12 fixé rigidement sur une potence et un second patin fémoral 13 coulissant sur ladite potence.

Une partie de la potence constituera donc une glissière 14 pour le patin fémoral mobile.

Pour des raisons d'encombrement, les patins fémoraux seront alors d'épaisseur réduite.

Dans une autre variante de réalisation (figure 6), le tenseur 10 comprenant une potence 11 et un patin fémoral 12 présente, de part et d'autre de la potence, à savoir au niveau de la fibre tendue et de la fibre comprimée, une jauge 7C de contrainte.

Cela permet de s'affranchir du point d'application de la force sur le patin et de la section du patin.

La potence présente des rainures 14 localisées au niveau des fibres tendues et comprimées afin d'y loger les jauges de contrainte et les fils qui vont les relier au dispositif de visualisation de la mesure.

Comme indiqué plus haut, l'ancillaire permet de préserver la balance ligamentaire par une mesure précise de la traction des ligaments.

La figure 6 montre un patin tibial 16 et la partie fémorale fixée sur la potence.

Un système expert analysera les valeurs produites par les jauges.

## Revendications

1. Ancillaire pour la préparation de la mise en place d'une prothèse de genou comprenant un patin fémoral (12) fixé rigidement sur une potence (11) et un second patin fémoral (13) coulissant sur ladite potence, **caractérisé en ce qu'**il comprend un capteur (7B) de force disposé entre le patin fixé et le patin coulissant.

2. Ancillaire pour la préparation de la mise en place d'une prothèse de genou selon la revendication 1 **caractérisé en ce qu'**une partie de la potence constitue une glissière (14) pour le patin fémoral mobile.

3. Ancillaire pour la préparation de la mise en place d'une prothèse de genou comprenant un tenseur (10) avec une potence (11) et un patin fémoral (12) **caractérisé en ce qu'**il présente, de part et d'autre de la potence, à savoir au niveau de la fibre tendue et de la fibre comprimée, une jauge (7C) de contrainte.

4. Ancillaire pour la préparation de la mise en place d'une prothèse de genou selon la revendication 3 **caractérisé en ce que** la potence présente des rainures (14) localisées au niveau des fibres tendues et comprimées afin d'y loger les jauges de contrainte et les fils qui vont les relier au dispositif de visualisation de la mesure.

## Patentansprüche

1. Hilfsmittel zur Vorbereitung des Einsetzens einer Knieprothese, umfassend, eine Femurplatte (12), die starr auf einer Stütze (11) befestigt ist und eine zweite Femurplatte (13), die sich auf der Stütze verschiebt, **dadurch gekennzeichnet, daß** sie einen zwischen der befestigten Platte und der sich verschiebenden Platte angeordneten Kraftsensor (7B) umfaßt.

2. Hilfsmittel zur Vorbereitung des Einsetzens einer Knieprothese nach Anspruch 1, **dadurch gekennzeichnet, daß** ein Teil der Stütze eine Gleitführung (14) für die bewegliche Femurplatte bildet.

3. Hilfsmittel zur Vorbereitung des Einsetzens einer Knieprothese, umfassend eine Spannvorrichtung (10) mit einer Stütze (11) und einer Femurplatte (12), **dadurch gekennzeichnet, daß** sie beiderseits der Stütze, nämlich auf der Ebene der gespannten Faser und der komprimierten Faser einen Dehnungsmeßstreifen (7C) umfaßt.

4. Hilfsmittel zur Vorbereitung des Einsetzens einer Knieprothese nach Anspruch 3, **dadurch gekennzeichnet, daß** die Stütze Rillen (14) aufweist, die auf der Ebene der gespannten und komprimierten Fasern angeordnet sind, um dort die Dehnungsmeßstreifen und die Fäden unterzubringen, die sie mit einer Vorrichtung zur Visualisierung der Messung verbinden.

## Claims

1. Ancillary for preparing for the fitting of a knee prosthesis comprising a femoral patten (12) rigidly fixed to a post (11) and a second femoral patten (13) gliding on the said post, **characterised in that** it includes a force transducer (7B) arranged between the fixed patten and the sliding patten.

2. Ancillary for preparing for the fitting of a knee prosthesis according to Claim 1, **characterised in that** a part of the post constitutes a slide (14) for the moving femoral patten.

3. Ancillary for preparing for the fitting of a knee prosthesis including a tensioner (10) with a post (11) and a femoral patten (12), **characterised in that** it has, on either side of the post, i.e. at the level of the stretched fibre and the compressed fibre, a strain gauge (7C).

4. Ancillary for preparing for the fitting of a knee prosthesis according to Claim 3, **characterised in that** the post has grooves (14), located at the level of the stretched and compressed fibres, in which to house the strain gauges and the wires which will connect them to the measurement display device.
